# EUROPEAN PATENT SPECIFICATION

(11) **EP 2 403 957 B1**
(45) Date of publication and mention of the grant of the patent: **08.01.2014**
(21) Application number: 10708817.1
(22) Date of filing: 03.03.2010
(51) Int. Cl.: C12Q 1/04, G01N 33/68, G06F 17/00, G06F 19/00, C12M 1/34

(54) **METHOD FOR IDENTIFYING GERMS IN A LIQUID MEDIUM**
VERFAHREN ZUR IDENTIFIZIERUNG VON KEIMEN IN EINEM FLÜSSIGEN MEDIUM
PROCÉDÉ D'IDENTIFICATION DE GERMES DANS UN MILIEU LIQUIDE

(30) Priority: 03.03.2009 FR 0900954
(43) Date of publication of application: 11.01.2012
(73) Proprietor: Assistance Publique-Hôpitaux de Paris (APHP), 75004 Paris (FR); Université Paris Descartes, 75270 Paris Cedex 06 (FR)
(72) Inventor: NASSIF, Xavier, F-75015 Paris (FR); FERRONI, Agnès, F-92140 Clamart (FR); DAUPHIN, Brunhilde, F-75009 Paris (FR); CARBONNELLE, Etienne, F-75015 Paris (FR); BERETTI, Jean-Luc, F-78340 Les Clayes sous Bois (FR)
(74) Representative: Peaucelle, Chantal
(86) International application number: PCT/IB2010/050913
(87) International publication number: WO 2010/100612

(56) References cited:
- WO-A1-95/15397
- WO-A1-2010/062349
- WO-A1-2010/062350
- WO-A1-2010/062351
- WO-A1-2010/062352
- WO-A1-2010/062354
- WO-A1-2010/062356
- WO-A2-01/92872
- WO-A2-02/21108
- WO-A2-2008/084409
- WO-A2-2011/006911
- FENSELAU C ET AL: "Characterization of intact microorganisms by MALDI mass spectrometry" MASS SPECTROMETRY REVIEWS, JOHN WILEY AND SONS, NEW YORK, NY, US, vol. 20, no. 4, 1 July 2001 (2001-07-01), pages 157-171, XP002516413 ISSN: 0277-7037 [retrieved on 2001-12-21]
- KEYS C J ET AL: "Compilation of a MALDI-TOF mass spectral database for the rapid screening and characterisation of bacteria implicated in human infectious diseases" INFECTION, GENETICS AND EVOLUTION, ELSEVIER, AMSTERDAM, NL, vol. 4, no. 3, 1 September 2004 (2004-09-01), pages 221-242, XP004533650 ISSN: 1567-1348
- CHEN EMILY I ET AL: "Optimization of mass spectrometry-compatible surfactants for shotgun proteomics." JOURNAL OF PROTEOME RESEARCH JUL 2007, vol. 6, no. 7, July 2007 (2007-07), pages 2529-2538, XP002532897 ISSN: 1535-3893 cited in the application
- CHEN EMILY I ET AL: "Comparisons of mass spectrometry compatible surfactants for global analysis of the mammalian brain proteome." ANALYTICAL CHEMISTRY 15 NOV 2008, vol. 80, no. 22, 15 November 2008 (2008-11-15), pages 8694-8701, XP002532898 ISSN: 1520-6882
- MEETANI M A ET AL: "MALDI Mass Spectrometry Analysis of High Molecular Weight Proteins from Whole Bacterial Cells: Pretreatment of Samples with Surfactants" JOURNAL OF THE AMERICAN SOCIETY FOR MASS SPECTROMETRY, ELSEVIER SCIENCE INC, US LNKD- DOI:10.1016/J.JASMS.2005.04.004, vol. 16, no. 9, 1 September 2005 (2005-09-01), pages 1422-1426, XP025302923 ISSN: 1044-0305 [retrieved on 2005-09-01]
- DEGAND NICOLAS ET AL: "Matrix-assisted laser desorption ionization-time of flight mass spectrometry for identification of nonfermenting gram-negative bacilli isolated from cystic fibrosis patients.", JOURNAL OF CLINICAL MICROBIOLOGY OCT 2008, vol. 46, no. 10, October 2008 (2008-10), pages 3361-3367, XP007921431, ISSN: 1098-660X
- Carol L. Nilsson: "Fingerprinting of Helicobacter pylori strains by matrix-assisted laser desorption/ionization mass spectrometric analysis", Rapid Communications in Mass Spectrometry, vol. 13, no. 11, 15 June 1999 (1999-06-15) , pages 1067-1071, XP055049776, ISSN: 0951-4198, DOI: 10.1002/(SICI)1097-0231(19990615)13:11<106 7::AID-RCM612>3.0.CO;2-N
- VORM, CHAIT, ROEPSTORFF: "Mass Spectrometry of Protein Samples Containing Detergents", 41TH ASMS CONFERENCE PROCEEDINGS, 1994, pages 621a-621b, XP009166172,

## Description

This invention relates to a method for identifying germs in a liquid medium.

A period of 24 to 48 hours is required to identify germs cultured in a biological fluid.

In fact, a culture of germs in a liquid medium must be subcultured on a solid medium,

This period of time is detrimental to the need to promptly initiate appropriate empirical antibiotic therapy before having the results of the antibiogram at hand.

Molecular biology techniques (PCR) applied directly to blood culture broths are faster; nevertheless, they take a minimum of about 3 h and require appropriate facilities and a degree of technical expertise.

WO 02/21108 (Large Scale Proteomics Corp. et al) relates to the detection and the characterization of micro-organisms comprising separating the micro-organisms from a mixture by 2D centrifugation.

The articles of Chen et al in Anal. Chem. 2008, 80,8694-8701 and J. Proteome Res; July 2007: 6(7); 2529-2538 disclose tension-active agents for mass spectrometry useful in proteomic.

Meetani and Voorhees, in J Am Soc Mass Spectrom, 2005, 16, 1422-1426, disclose a MALDI-TDF MS analysis, comprising the use of nonionic and zwitterionic surfactants for detection of high molecular weight proteins from cultured bacterial isolates.

WO 2010/062352 and WO 2010/062354 of Biomerieux Inc. et al, which fall under Art.54(3)EPC, disclose methods for the isolation and identification of microorganisms in a test sample, which may comprise a lysis step for lysing non-microorganism cells that may be present in the test sample, followed by a subsequent separation step.

Nilsson, in Rapid Communications in Mass Spectrometry 13, 1067-1071 (1999), discloses the fingerprinting of *Helicobacter pylori* by MALDI-TOF. Lysates and extracts of different strains were examined and the effects of solubilising analytes with n-octyl-β-D-glucopyranoside were explored.

Vorm et al, The Rockfeller University, N.Y., New York 10021, have presented at the 41^{st} ASMS Conference on Mass Spectrometry, 1994, results concerning mass spectrometry of protein samples (peptides mixture from tryptic digest) containing detergents.

WO 95/15397 of Gordon Dorn discloses a method for improving quantitative recovery of microorganisms from specimens containing blood components wherein purified saponin is combined with sodium polyanethol sulfonate to provide increased quantitation of microorganisms.

The inventors' work focused on seeking means for overcoming the drawbacks of the prior art. The inventors sought to adapt the MALDI-TOF MS (Matrix-Assisted Laser Desorption/Ionisation Time-of-Flight Mass Spectrometry) technique to the identification of germs in a liquid medium, making the most of their expertise in both bacteriology and the analysis of microorganisms by mass spectrometry.

It is known that bacteria which have been grown on a solid medium can currently be identified by MALDI-TOF MS thanks to the specific profiles of species which have been obtained directly from colonies.

The inventors have found that this technique can be used by appropriately treating the germs contained in a liquid sample or biological sample, whether or not this sample has been enriched in a liquid medium.

Accordingly, the object of the invention is to provide a new method for identifying germs in a liquid medium.

The method of the invention for identifying germs in a sample of liquid medium comprises the steps of:
- adding saponin to the liquid medium containing components of a host infected by germs, i.e. cellular components and proteins from the extracellular environment, to release the germs from the components of the infected host without degrading the membranes of the germs ;
- recovering the germs from the liquid medium by differential centrifugation ;
- analysing the germ pellet by MALDI-TOF MS, followed by comparing the results from germ databases.

This technique has the advantage of being rapid: it produces a gain in time of at least 24 h in the identification process by avoiding subculture on a solid medium, so that appropriate antibiotic therapy can thus be initiated without loss of time. Furthermore, the technique is easy to implement.

The detergent, i.e saponin is added to the liquid medium to a final concentration of at most 5%, more preferably of 0.5-5%, most preferably of 1%.

According to other conditions, the detergent is added to a final concentration of 1 to 5%, preferably 2%.

Advantageously, contact time between the liquid containing the germs to be analysed and the detergent is at most 8 min. The mixture is centrifuged, advantageously after adding distilled water.

Appropriate centrifugation conditions are 20,000 rpm at most, particularly 10,000-15,000 rpm, for 15 min at most, preferably for approximately 1-15 min, and more particularly for approximately 2 min.

According to other conditions, the centrifugation is performed at 8000 t/min at most, preferably 3000-5000 t/min, during 15 min at most, preferably about 5-15 min, and more particularly during about 5 min.

The supernatant is then discarded and the pellet is taken up in distilled water and centrifuged, advantageously under the conditions defined above. The pellet is recovered and used for the MALDI-TOF MS analysis. The results obtained are compared with the databases.

Liquids used in the method according to the invention include, for example, blood cultures from patients positive for a particular germ, or urine, provided the infection is suspected to be monomicrobial. This can be detected either directly from the biological fluid, or after enrichment in a liquid medium when the inoculum is sparse.

The spectrum is advantageously analysed according to the technique described in international application WO2008/084409, filed on 8 January 2008, for 'Means for identifying a germ isolated from a clinical sample'.

Various types of germs can be identified such as bacteria, yeasts, fungi or, if appropriate, filamentous fungi.

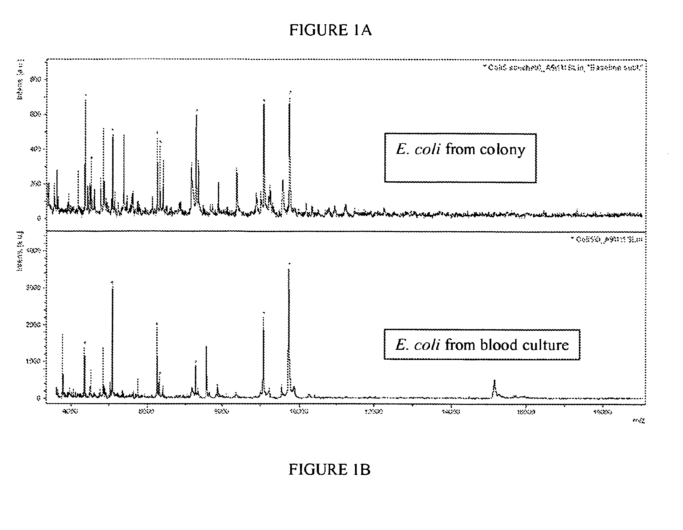

Other features and advantages of the invention are set forth the following examples, in which reference is made to Figs. 1A and 1B which are the MALDI-TOF MS graphs obtained for germs isolated from blood cultures and one isolated colony, respectively.Common peaks between the two spectra are marked by "*". These peaks are specific of the *E. coli* species (a.u : arb. unit (absorption)).

### Material and methods

### Blood and fluids cultures

Preliminary tests used negative blood culture flasks without charcoal (bioMérieux, Marcy l'Etoile, France). They were artificially contaminated with 10⁴ cells of commonly isolated pathogens (table1), then placed in the automated blood culture apparatus Bact/Alert (bioMerieux, Marcy-l'Etoile, France) until detection of positivity. In addition, different pathological fluids from patients were tested: positive blood cultures (table 2 and 3) or different fluids spiked into blood culture flasks (table 4).

Two aliquots from the blood culture bottle were taken. The first aliquot was taken for MALDI-TOF-MS processing. The second was used for Gram staining, antibiotic suceptibility testing, and appropriate subcultures for microbiological identification using conventional microbiological techniques.

### MALDI-TOF-MS

Two hundred microliters of the positive blood culture broth (or 1 ml of enrichment liquids) were transferred into a plastic tube containing 40 µl (or 200µl for enrichment liquids) of a solution of 5% saponin to release intracellular bacteria. After 5 minutes incubation, distilled water was added up to 1.5 ml and 2 consecutive washes in distilled water were performed at 16600 g for one minute. The supernatant was discarded and 5 µl of 10% trifluoroacetic acid was added to the pellet. One µl of this mixture was spotted (2 wells/sample) onto a MALDI sample target (Bruker Daltonics, Bremen, Germany) and allowed to dry at room temperature. One microliter of absolute ethanol was then added to each well, and the mixture allowed to dry. One µl of matrix solution DHB (2,5-dihydroxybenzoic acid, 80 mg/ml, 30% acetonitrile, 0.1% trifluoroacetic acid) was then added and allowed to co-crystallize with the sample. Samples were processed in the MALDI-TOF-MS spectrometer (Microflex, Bruker Daltonics) with the Flex Control software (Bruker Daltonics). Positive ions were extracted with an accelerating voltage of 20 kV in linear mode. Each spectrum was the sum of the ions obtained from 400 laser shots performed automatically on different regions of the same well. The spectra were analyzed in an m/z range of 3640 to 2000, and compared with those of a reference database (Andromas@SAS, Paris, France). This database has been engineered as previously described and encompasses the pathogens encountered in human pathology (1, 2). The identification of the tested strain corresponds to the species of the reference strain having the best match in the database. The analysis takes also into account the difference between the first two species having the best matches with the reference database. The species identification was considered to be valid if, for one of the two sample deposits, the percentage of matched peaks was at least 60% of that of the first species proposed in the database, after analysis by the Andromas@ software (Andromas SAS), and if the difference between the first two species having the best match in the database is at least 10%. If the latter condition was not fulfilled, the identification was considered as being correct at the level of the group/genus/family if the first two matches belonged to the same group/genus/family of bacteria. In all other cases, the results were considered as irrelevant. It should be pointed out that most unreliable identifications were due to poor quality spectra. When the blood cultures contained several bacterial species as seen on Gram staining, databases specific for Gram negative bacilli and/or Gram positive cocci were used.

### Results

### 1) Identification of germs spiked in blood culture flasks

In order to determine whether an accurate identification of pathogens could be obtained from bacteria grown in liquid media, pilot experiments were first performed using blood culture bottles spiked with commonly isolated pathogens. Figures 1A and 1B show an example of spectrum obtained with *Escherichia coli* grown in blood culture bottles, compared to that of the same strain obtained from an isolated colony. A total of 292 bacterial strains and 20 *Candida* species were spiked in blood culture bottles. Results are shown in Table 1.

**Table 1. Microbiological identification by MALDI-TOF-MS of blood cultures spiked with different species**

| **Species identification obtained from subcultures (biochemical or molecular techniques)** | **Tested samples** | **Identification** | | | | |
|---|---|---|---|---|---|---|
| | | **Species** | **Group** | **Genus** | **Family** | **Unacceptable profiles** |
| ***Staphylococcus*** | | | | | | |
| *S. aureus* | 42 | 42 | | | | |
| *S. epidermidis* | 7 | 7 | | | | |
| *S. haemolyticus* | 7 | 6 | 1 (CNS group^{a}) | | | |
| *S. hominis* | 3 | 3 | | | | |
| *S. warneri* | 5 | 5 | | | | |
| *S. lugdunensis* | 2 | 2 | | | | |
| *Micrococcus luteus* | 5 | 3 | 1 (CNS group^{a}) | | 1 | |
| ***Streptococcacae*** | | | | | | |
| *S. mitis* | 6 | | 6 (*pneumoniae*/*mitis* group) | | | |
| *S. pneumoniae* | 6 | | 6 (*pneumoniaelmitis* group) | | | |
| *S. gordonii* | 4 | | 4 (oral *streptococci* group^{b}) | | | |
| *Enterococcus faecalis* | 14 | 14 | | | | |
| ***Gram negative non fermenting bacilli*** | | | | | | |
| *P. aeruginosa* | 39 | 38 | | | 1 | |
| *S. maltophilia* | 20 | 18 | | | | 2 |
| *A. baumanii* | 15 | 15 | | | | |
| ***Enterobacteriacae*** | | | | | | |
| *E. coli* | 20 | 19 | | | | 1 |
| *E. cloacae 18* | | 13 | | | 5 | |
| *Citrobacter group* | 16 | 14 | | | 1 | 1 |
| *freundii* | | | | | | |
| *K. oxytoca* | 17 | 17 | | | | |
| *K. pneumoniae* | 20 | 18 | 1 (KES group) | | | 1 |
| *P. mirabilis* | 18 | 17 | | 1 | | |
| ***H. influenzae Anaerobes*** | 2 | 2 | | | | |
| *C. perfringens* | 2 | 2 | | | | |
| *F. necrophorum* | 3 | 3 | | | | |
| *B. fragilis* | 1 | 1 | | | | |
| ***C. albicans*** | 10 | 10 | | | | |
| **Other *Candida* species** | 10 | 10 | | | | |
| **Total** | 312 | 279 | 19 | | 1 8 | 5 |

| | | | | | | |
|---|---|---|---|---|---|---|
| ^{a} CNS group : coagulase negative *Staphylococcus* ^{b} Oral *Streptococci* group : group including all oral species of *Streptococci* excepted the *S. milleri group* | | | | | | |

Of the 307 interpretable spectra (98%), MALDI-TOF-MS allowed a good identification at the species, group, genus and family level in 89%, 6%, 0.4% and 2.6 % of cases, respectively. It should be pointed out that MALDI-TOF-MS allowed differentiation of coagulase negative *Staphylococci* (CNS) from *Staphylococcus aureus* in 100% of cases.

### 2) Identification of positive blood cultures from patients Among the 388 positive blood cultures included in this study, 373 were monomicrobial (table 2).

**Table 2. Direct bacterial identification by MALDI-TOF-MS in monobacterial blood cultures from patients**

| **Species identification obtained from subcultures (biochemical or molecular techniques)** | **Tested samples** | **Identification** | | | | |
|---|---|---|---|---|---|---|
| | | **Species** | **Group** | **Genus** | **Family** | **Unacceptable profiles** |
| ***Staphylococcus*** | | | | | | |
| *S. epidermidis* | 121 | 118 | 3 (group CNS^{a}) | | | |
| *S. haemolyticus* | 3 | 3 | | | | |
| *S. hominis* | 20 | 20 | | | | |
| *S. pasteuri* | 1 | 1 | | | | |
| *S. capitis* | 3 | 2 | 1 (CNS group ^{a}) | | | |
| *S. aureus* | 43 | 42 | | | | 1 |
| *S. lugdunensis* | 1 | 1 | | | | |
| *Micrococcus luteus* | 1 | 1 | | | | |
| ***Streptococccae*** | | | | | | |
| *S. pyogenes* | 8 | 5 | 3 (*pyogenes*/*dysgalactiaegroup*^{b}) | | | |
| *S. mitis* | 8 | | 8 (*pneumoniae*/*mitis* group) | | | |
| *S. pneumoniae* | 1 | | 1 (*pneumoniae*/*mitis* group) | | | |
| *S. gordonii* | 1 | | 1 (oral *streptococci* group^{c}) | | | |
| *S. pasteurianus* | 1 | | | | | 1 |
| *S. salivarius* | 1 | | 1 (oral *streptococci* group^{c}) | | | |
| *S. oralis* | 2 | 2 | | | | |
| *E. faecalis* | 6 | 3 | | 2 | 1 | |
| *E. faecium* | 2 | 1 | | | 1 | |
| ***Gram negative non fermenting bacilli*** | | | | | | |
| *P. aeruginosa* | 20 | 17 | | | | 3 |
| *S. maltophilia* | 4 | 4 | | | | |
| *Achromobacter xylosoacydans* | 2 | 2 | | | | |
| *B. cenocepacia* | 1 | | 1 (*B. cepacia*/*cenocepacia* group) | | | |
| *P. oryzihabitans* | 1 | 1 | | | | |
| ***Enterobacteriacae*** | | | | | | |
| *E. coli* | 41 | 40 | | | | 1 |
| *E. cloacae* | 24 | 24 | | | | |
| *Cirobacter group freundii* | 9 | 6 | | | 1 | 2 |
| *E. aerogenes* | 7 | 5 | 1 (KES group) | | 1 | |
| *K. oxytoca* | 6 | 6 | | | | |
| *K. pneumoniae* | 10 | 10 | | | | |
| *P. mirabilis* | 8 | 8 | | | | |
| *S. marcescens* | 2 | 2 | | | | |
| *Salmonella enterica* | 3 | 3 | | | | |
| ***H. influenzae*** | 1 | 1 | | | | |
| ***C**. **albicans*** | 11 | 11 | | | | |
| **Total** | 373 | 339 | 20 | | 2 4 | 8 |

| | | | | | | |
|---|---|---|---|---|---|---|
| ^{a} CNS group : coagulase negative *Staphylococcus* ^{b} *pyogenes*/*dysgalactiaegroup*: no differentiation between *S. pyogenes* and *S. dysgalactiae* ^{c} Oral *Streptococci* group : group including all oral species of *Streptococci* exepted the *S. milleri group* | | | | | | |

Using MALDI-TOF-MS as described in the material and methods section an interpretable identification was obtained in 98% of cases. These results were concordant with those obtained by classical methods at the species, group, genus /family levels in 91%, 5%, and 2% of cases, respectively.

In addition, 15 blood cultures from patients containing mixed bacteria were tested (table 3).

**Table 3. Direct identification by MALDITOF MS of blood culture containing ≥ 2 germs**

| **Gram** | **Isolated microorganisms** | **Number** | **Identification** | | |
|---|---|---|---|---|---|
| | | | **General database** | **Gram negative *Bacilli* database** | **Gram positive *Cocci* database** |
| Gram positive cocci and Gram negative bacilli | *E. coli* | | | | |
| | *E. faecalis* | 1 | *E. coli* | *E. coli* | *E. faecalis* |
| | *S. epidermidis* | | | | |
| | *A. baumanii, S. mitis* | 2 | *A. baumanii* | *A. baumanii* | *Streptococcus* group *pneumo*/*mitis* |
| | *E. faecalis* + *E. coli* | I | *E. faecalis* | 0 | *E. faecalis* |
| | *S. aureus* | 4 | *S. aureus* (4/4) | *P. mirabilis (2*/*4)* | *S. aureus* (4/4) |
| | *P. mirabilis* | | | | |
| | *P. aeruginosa* + *S. epidermidis* | 1 | *P. aeruginosa*/ *S. epidermidis* | *P. aeruginosa* | *S. epidermidis* |
| Gram negative bacilli | *E. coli* + *M. morganii* | 1 | *E. coli* | *E. coli* | NA |
| | *E. coli* | 1 | *E. coli* | *E. coli* | NA |
| | *K. pneumoniae* | | | | |
| | *E. coli* + *P. mirabilis* | 1 | *E. coli* | *E. coli* | NA |
| | *K. pneumoniae* + *E. cloacae* | 1 | KES^{a} | KES^{a} | NA |
| Gram positive cocci | *E. faecalis* + *S. aureus* | 1 | *E. faecalis* | NA | *E. faecalis* |
| | *S. haemolyticus* + *S. hominis* | I | *S. hominis* | NA | *S. hominis* |

| | | | | | |
|---|---|---|---|---|---|
| ^{a}Group *Klebsiella-Enterobacter-Serratia* NA : non applicable | | | | | |

Using the database, either only one of the pathogens present in the mixture was detected, or two pathogens were detected at the same score. When Gram positive cocci and Gram negative bacilli were detected on the Gram staining, the identification was improved in 6 out of 9 cases by using a database containing species specific spectra of Gram positive cocci or Gram negative bacilli, respectively.

### 3) Identification of positive enrichment fluids from blood cultures flasks :

46 fluids grown in blood culture broths were included. The results are given in a below (table 4).

**Table 4. Direct bacterial identification by MALDI-TOF-MS in enrichment cultures from patients**

| **Species identification obtained from subcultures (biochemical or molecular techniques)** | **tested samples** | **identification** | | |
|---|---|---|---|---|
| | | **Species** | **Group** | **Genus** |
| **Graft conservation liquids** | | | | |
| *E. coli* | 3 | 2 | 1 (*Shigella* /*E*. *coli*)^{a} | |
| *H. alvei* | 2 | 2 | | |
| *S. epidermidis* | 7 | 7 | | |
| *S. warneri* | 2 | 2 | | |
| *S. cohnii* | 1 | 1 | | |

| **Articular fluids , bone ponctions, deep abscesses** | | | | |
|---|---|---|---|---|
| *S. aureus* | 16 | 16 | | |
| *S. epidermidis* | 5 | 5 | | |
| *S. capitis* | 1 | 1 | | |
| *S. lugdunensis* | 1 | 1 | | |
| *E. coli* | 4 | 4 | | |
| *E. cloacae* | 1 | 0 | | 1 |
| *P. aeruginosa* | 2 | 2 | | |
| *S. pyogenes* | 1 | 1 | | |
| | 46 | 44 | 1 | 1 |

| | | | | |
|---|---|---|---|---|
| ^{a}*Shigella*/*coli* : no differentiation between *Shigella sp* and *E. coli* | | | | |

All spectra were interpretable, and the obtained identification was concordant to that obtained by classical methods at the species, group and genus levels in 96%, 2% and 2% of cases, respectively.

### 4) Results on urines

10 urines were tested in 9 cases E.coli was identified and in 1 case a *S. saprophyticus* strain was identified.

The identifications were correct.

The method of the invention can be directly used on biological samples if the germs concentration is sufficient and the infection is a microbial infection.

### 5) Time to diagnosis

It should be pointed out that each positive blood culture was treated as soon as it was detected positive. The time required between the Bact/Alert alarm and the germ identification, including Gram staining performed during the incubation of detergent, was 20 min.

### Discussion

The sensitivity and accuracy of pathogen detection by MALDI-TOF-MS applied directly from Bact-Alert bottles was evaluated. This study enables a rapid (20 min) and reliable identification of the vast majority of microorganisms isolated in blood or fluids cultures. A rapid and accurate diagnosis diminishes the use of inadequate and broad-spectrum antibiotics, thereby improving outcome and reducing the potential development of resistance and possible side effects. Identification of microorganisms in blood cultures by MALDI-TOF-MS dramatically extends the influence of the result of the Gram staining on clinical management. In particular, among the Gram-negative bacilli, the differentiation of *Enterobacteriacae* from *Pseudomonas* or *Acinetobacter* only 20 min after the blood culture growth will allow a more appropriate treatment pending the results of susceptibility testing. Similarly, the possibility to obtain an immediate diagnosis of *S. aureus* is of major clinical consequence. Fast differentiation of *S. aureus* from CNS should help the clinician to discriminate a serious infection from a possible contamination.

### References

1. Carbonnelle, E., J. L. Beretti, S. Cottyn, G. Quesne, P. Berche, X. Nassif, and A. Ferroni. 2007. Rapid identification of Staphylococci isolated in clinical microbiology laboratories by matrix-assisted laser desorption ionization-time of flight mass spectrometry. J Clin Microbiol 45:2156-2161.
2. Degand, N., E. Carbonnelle, B. Dauphin, J. L. Beretti, M. Le Bourgeois, I. Sermet-Gaudelus, C. Segonds, P. Berche, X. Nassif, and A. Ferroni. 2008. Matrix-assisted laser desorption ionization-time of flight mass spectrometry for identification of nonfermenting gram-negative bacilli isolated from cystic fibrosis patients. J Clin Microbiol 46:3361-3367.

## Claims

1. A method for identifying germs in a sample of liquid medium, comprising:
- adding saponin to the liquid medium containing components of a host infected by germs, i.e. cellular components and proteins from the extracellular environment, to release the germs from the components of the infected host without degrading the membranes of the germs ;
- recovering the germs from the liquid medium by differential centrifugation;
- analysing the germ pellet by MALDI-TOF MS, followed by comparing the results from germ databases.

2. The method of claim 1, wherein the detergent is added to the liquid medium to a final concentration of at most 5%.

3. The method of claim 2, wherein said concentration is 1-5%.

4. The method of claim 3, wherein said concentration is 2%.

5. The method of anyone of claims 1 to 4, wherein the contact time between the liquid containing the germs to be analysed and the detergent is at most 8 min.

6. The method of anyone of claims 1 to 5, wherein the liquids used are blood cultures from patients positive for a particular germ, or urine, provided the infection is suspected to be monomicrobial.

## Patentansprüche

1. Verfahren zum Identifizieren von Keimen in einem flüssigen Medium, umfassend:
- Hinzufügen von Saponin zu dem flüssigen Medium, das Komponenten eines von Keimen infizierten Wirts, d.h. zelluläre Komponenten und Proteine aus der extrazellulären Umgebung, enthält, wobei die Keime aus den Komponenten des infizierten Wirts freigesetzt werden, ohne die Membranen der Keime abzubauen;
- Gewinnen der Keime aus dem flüssigen Medium durch differentielle Zentrifugation;
- Analysieren des Keimsediments durch MALDI-TOF-MS und anschließend Vergleichen der Ergebnisse mit Keimdatenbanken.

2. Verfahren gemäß Anspruch 1, wobei das Detergens bis zu einer Endkonzentration von höchstens 5% zu dem flüssigen Medium gegeben wird.

3. Verfahren gemäß Anspruch 2, wobei die Konzentration 1-5% beträgt.

4. Verfahren gemäß Anspruch 3, wobei die Konzentration 2% beträgt.

5. Verfahren gemäß einem der Ansprüche 1 bis 4, wobei die Kontaktzeit zwischen der Flüssigkeit, die die zu analysierenden Keime enthält, und dem Detergens höchstens 8 min beträgt.

6. Verfahren gemäß einem der Ansprüche 1 bis 5, wobei es sich bei den verwendeten Flüssigkeiten um Blutkulturen von Patienten, die auf einen bestimmten Keim positiv getestet wurden, oder Urin handelt, mit der Maßgabe, dass angenommen wird, dass die Infektion monomikrobiell ist.

## Revendications

1. Méthode d'identification de germes dans un milieu liquide **caractérisée par**
- l'addition de saponine au milieu liquide renfermant des éléments d'un hôte infecté par des germes, à savoir des composants cellulaires et des protéines du milieu extracellulaire, pour libérer les germes des éléments de l'hôte infecté, sans altérer les membranes des germes,
- la récupération des germes du milieu liquide par centrifugation différentielle ;
- l'analyse par spectrométrie MALDI-TOF-MS sur le culot de germes, suivie d'une comparaison des résultats avec des bases de données de germes.

2. Méthode selon la revendication 1 , **caractérisée en ce que** le détergent est ajouté au milieu liquide jusqu'à une concentration de 5% au plus..

3. Méthode selon la revendication 2, **caractérisée en ce que** la concentration est de 1-5%.

4. Méthode selon la revendication 3, **caractérisée en ce que** la concentration est de 2%.

5. Méthode selon l'une quelconque des revendications 1 à 4, **caractérisée en ce que** le temps de contact avec le liquide renfermant les germes à analyser et le détergent est de 8 min au plus.

6. Méthode selon l'une quelconque des revendications 1 à 5, **caractérisée en ce que** les liquides utilisés sont des hémocultures de malades positif à un germe donné, ou des urines dans la mesure où l'infection est suspectée monomicrobienne.
